# EUROPEAN PATENT SPECIFICATION

(11) **EP 2 897 539 B1**
(45) Date of publication and mention of the grant of the patent: **21.12.2016**
(21) Application number: 13774847.1
(22) Date of filing: 19.09.2013
(51) Int. Cl.: H01L 21/62, A61B 17/00, A61B 17/32, A61B 17/3211

(54) **MICROMACHINED ULTRASONIC SCALPEL WITH EMBEDDED PIEZOELECTRIC ACTUATOR**
MIKROGEFERTIGTES ULTRASCHALLSKALPELL MIT EINEM INTEGRIERTEN PIEZOELEKTRISCHEN AKTUATOR
SCALPEL À ULTRASONS MICRO-USINÉ AYANT UN ACTIONNEUR PIÉZOÉLECTRIQUE INTÉGRÉ

(30) Priority: 19.09.2012 US 201213622921
(43) Date of publication of application: 29.07.2015
(73) Proprietor: Ethicon Endo-Surgery, LLC, 00969 Guaynabo (PR)
(72) Inventor: DIETZ, Timothy G., Terrace Park, Ohio 45174 (US); TARDY, Yanik, 2206 Geneveys-sur-Coffrane (CH); BURGER, Juergen, 2563 Ipsach (CH); MARGAIRAZ, Philippe, 2300 La Chaux-de-Fonds (CH); BORK, Toralf, 2073 Enges (DE)
(74) Representative: Carpmaels & Ransford LLP
(86) International application number: PCT/US2013/060532
(87) International publication number: WO 2014/047241

(56) References cited:
- WO-A1-02/062241
- WO-A1-2008/154338
- WO-A2-2011/020097
- US-A1- 2002 091 404

## Description

### FIELD

The invention relates to ultrasonic surgical instruments and, more particularly, to transducer and waveguide assemblies for ultrasonic surgical scalpels and similar instruments having ultrasonically powered end effectors.

### BACKGROUND

Ultrasonic surgical scalpels and similar instruments for the dissection and/or coagulation of patient tissue typically comprise an ultrasonic transducer assembly, a waveguide assembly, and a surgical end effector. The ultrasonic transducer assembly generally comprises a piezoelectric transducer element compressed between a pair of end masses, with the fore-end mass being configured as an acoustic horn to create acoustic gain between the piezoelectric transducer element and the waveguide assembly. In one known example, the end masses are disposed at the opposite ends of a shaft, and the piezoelectric transducer element comprises a plurality of annular piezoelectric transducer disks disposed along the shaft, with the plurality of disks being compressed, for example, by tightening a threaded connection between the shaft and at least one of the end masses. The piezoelectric transducer element is subsequently powered to establish at least one standing wave or mode of vibration (which may include, without limitation, a longitudinal mode of vibration, a lateral mode of vibration, a torsional mode of vibration, and combinations thereof) which propagates through the shaft and acoustic horn, through the waveguide assembly, and into the ultrasonically powered end effector for application to patient tissue. Exemplary end effectors powered by such devices include ultrasonically vibrated surgical scalpels for the dissection of patient tissue and ultrasonically vibrated clamp devices for the apposition and cauterization of patient tissue.

The applicant-assignee has recently disclosed various ultrasonic surgical instruments which include transducer elements affixed to longitudinally elongated, generally planar waveguides. See U.S. Patent Publication Nos. 2011/0040212 A1 and 2011/0040213 A1, both filed August 16, 2010. Such instruments may be constructed upon, for example, a silicon wafer, with transduction and resonator portions taking the place of the shaft and end masses of the aforedescribed ultrasonic transducer assembly. However, the transducer elements in such devices cannot be secured between and compressed by adjustable end masses like the ones in the aforedescribed ultrasonic transducer assembly. Consequently, the applicant and its associates have continued to seek and to develop improved constructions for these novel and essentially monolithic transducer-supporting waveguides.

WO 2011/020097 discloses a silicon waveguide for an ultrasonic surgical instrument. The waveguide is a longitudinally elongated, planar waveguide with a first resonator or proximal end portion, a transduction portion, and a second resonator or distal end portion. A piezoelectric or electrostrictive ceramic transducer is directly bonded to both sides of the transduction portion. The transduction portion includes at least one aperture which is filled by a bridging portion of the monolithic transducer.

WO 2008/154338 discloses an APA transducer driven surgical cutting device comprising a body, an APA transducer, and a blade neck attached to the transducer. The transducer is a flextensional transducer assembly including a cell housed within a flexible frame.

WO 02/062241 discloses an ultrasonic surgical instrument with an ultrasonic member comprising a piezoelectric laminate structure which includes a frame, a resonant structure, and a transducer. The transducer includes a pair of crystals separated by a silicon plate. A bonding agent or process is used to fasten the crystals to the plate. The resonant structure includes first and second resonant members which together define a cavity for receiving the transducer.

### SUMMARY

The present invention provides an ultrasonic core for an ultrasonic surgical instrument as recited in independent claim 1 and its dependent claims. The transducer element is at least partially embedded within the waveguide.

A second aspect of the invention is a method for assembling an ultrasonic core as recited in independent claim 9 and its dependent claims. The core may be the core of the aforementioned embodiments.

Also disclosed is an ultrasonic core for an ultrasonic surgical instrument. The ultrasonic core includes a longitudinally elongated, generally planarwaveguide; a transducer element secured to the waveguide, and a clamp mechanism. The clamp mechanism includes a base disposed proximally from the proximal end of the waveguide, a pair of restraining arms projecting distally from the base and configured so as to mutually oppose one another across a channel housing the waveguide, and a clamp arm projecting distally from the base between the pair of restraining arms. The base and the clamp arm are mechanically engaged with one another so as to permit the distal end of the clamp arm to be adjustably and securely positioned within the channel, and each restraining arm includes a mount which engages the waveguide at a node positioned distally from the transducer element. The clamp arm may engage a proximal end of the waveguide or a proximal end of a transducer element secured to a proximal end of the waveguide.

Also disclosed is an ultrasonic handpiece for an ultrasonic surgical instrument. The ultrasonic handpiece includes a longitudinally elongated, generally planar waveguide, a transducer element secured to the waveguide, a housing surrounding at least the transducer element, and a clamp mechanism secured to the housing proximate the transducer element. The clamp mechanism engages the transducer element at a transducer node, and both the clamp mechanism and the transducer element include complementary electrical contacts for applying a drive current to the transducer element.

Also disclosed is an ultrasonic core optionally including an surgical scalpel portion. The ultrasonic core includes a longitudinally elongated, generally planar silicon waveguide having a generally planar transduction portion, with at least one transducer element secured to the generally planar transduction portion, and a wedge-shaped acoustic horn portion including an inclined side surface. The ultrasonic core is characterized in that the inclined side surface is oriented along the {1,1,1} crystallographic plane of the silicon material. The wedge-shaped acoustic horn portion may include a unitary surgical scalpel portion as a part of the inclined side surface.

Also disclosed is a method of manufacturing a silicon waveguide for an ultrasonic surgical instrument where a wedge-shaped distal portion of the waveguide includes an inclined side surface oriented along a {1,1,1} crystallographic plane of the silicon material. The method includes the ordered steps of: (a) obtaining a silicon wafer cut so as to have the {1,1,1} crystallographic plane disposed at a non-zero acute angle with respect to a face of the wafer; (b) growing a thermal oxide coating upon the wafer; (c) applying a photoresist coating to one face of the wafer; (d) exposing the applied photoresist to a light shown through a photomask bearing a pattern representative of the inclined side surface of the waveguide; (e) performing an oxide etch upon the thermal oxide coating exposed by the light-induced destruction of the photoresist coating and then removing the residual photoresist coating; (f) performing a hydroxide etch upon the silicon exposed by the oxide etch of the thermal oxide coating until the silicon is removed to a predetermined maximum depth; and (g) dicing the silicon wafer to create a longitudinally elongated, generally planar waveguide having at least a wedge-shaped acoustic horn including the inclined side surface. Additional steps may be performed prior to the dicing of the silicon wafer to create a longitudinally elongated, generally planar waveguide having a wedge-shaped acoustic horn portion and a unitary surgical scalpel portion where both portions include the inclined side surface.

Other aspects of the disclosed ultrasonic cores, handpieces, and surgical instruments will become apparent from the following description, the accompanying drawings, and the appended claims.

### BRIEF DESCRIPTION OF THE DRAWINGS

Fig. 1 is a plan view of a waveguide for a first embodiment of an ultrasonic core.
Fig. 2 is a sectional view of the waveguide of Fig. 1.
Fig. 3 is a plan view of a first embodiment of an ultrasonic core including a transducer element configured as a "Langevin stack."
Fig. 4 is a sectional view of the ultrasonic core of Fig. 3.
Fig. 5 is a detail view of the interface between the transducer element and the waveguide of the device of Figs. 3 and 4.
Fig. 6 is a plan view of a waveguide for a second embodiment of an ultrasonic core.
Fig. 7 is a sectional view of the waveguide of Fig. 6.
Fig. 8 is a sectional view of a first example of a second embodiment of an ultrasonic core.
Fig. 9 is a sectional view of a second example of a second embodiment of an ultrasonic core.
Fig. 10 is an illustration of a method for assembling the ultrasonic cores of the first and second embodiments which specifically illustrates the assembly of an exemplary device of the first embodiment.
Fig. 11 is plan view of a first example of a third embodiment of an ultrasonic core which includes a clamp mechanism for preconstraining a transducer element.
Fig. 12 is a plan view of a second example of an ultrasonic core which includes a clamp mechanism for preconstraining a transducer element
Fig. 13 is a partial sectional view of a third example of a third embodiment of an ultrasonic core which includes a clamp mechanism for preconstraining a transducer element.
Fig. 14 is a partial sectional view of a fourth example of an ultrasonic core which includes a clamp mechanism for preconstraining a transducer element.
Fig. 15 is a sectional view of an ultrasonic core and handpiece.
Fig. 16 is a sectional view of an ultrasonic core and handpiece.
Fig. 17 is a plan view of an ultrasonic core and surgical scalpel.
Fig. 18 is a sectional view of the ultrasonic core and surgical scalpel of Fig. 17.
Figs. 19A-D illustrate a method for manufacturing a waveguide for the devices of the invention

Exemplary illustrations are sectional views of the silicon wafer unless otherwise indicated.

### DETAILED DESCRIPTION

Before explaining the several embodiments in detail, it should be noted that the embodiments and expressions are not limited in their application or use to the details of construction and arrangement of parts illustrated in the accompanying drawings and description. The illustrative embodiments and expressions may be implemented or incorporated in other embodiments, expressions, variations, and modifications, and may be practiced or carried out in various ways. Furthermore, unless otherwise indicated, the terms and expressions employed herein have been chosen for the purpose of describing the illustrative embodiments for the convenience of the reader, and are not for the purpose of limiting the invention.

It is further understood that any one or more of the following-described embodiments, expressions, examples, etc. may be combined with any one or more of the other following-described embodiments, expressions, examples, etc. Such modifications and variations are intended to be included within the scope of the claims.

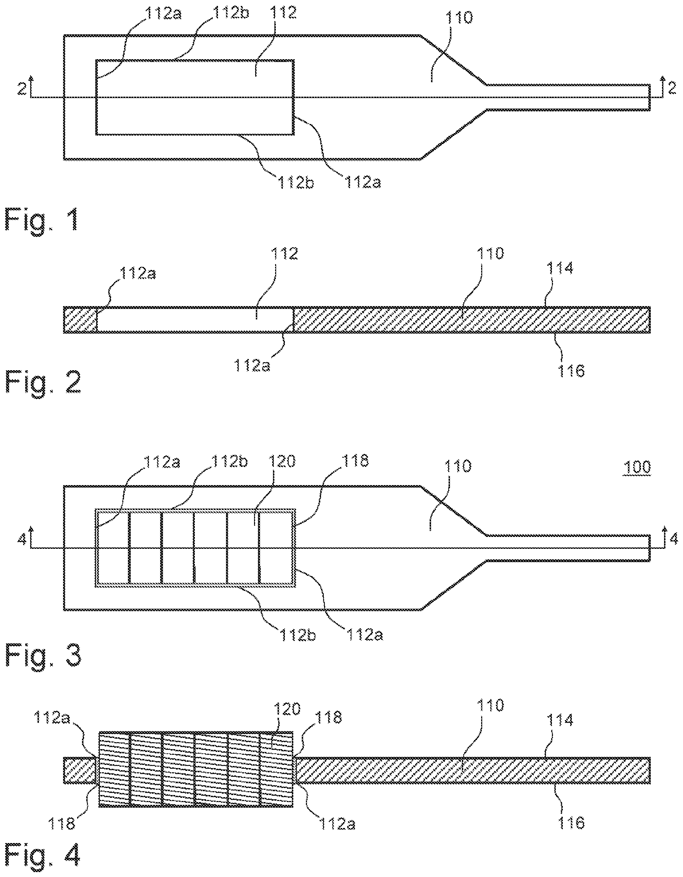

A first embodiment is shown in Figures 1-5. The first embodiment is an ultrasonic core 100 for an ultrasonic surgical instrument, and includes a longitudinally elongated, generally planar waveguide 110. The waveguide material is preferably a single crystal or polycrystalline material, principally silicon, although germanium, diamond, or sapphire may also be used. In alternate constructions, the generally planar waveguide 110 may instead be manufactured from glass, ceramic, titanium, stainless steel, or aluminum. Although typically illustrated as having only a single layer, it will be understood that the waveguide may be a laminated structure including a plurality of planar layers. For example, for a silicon waveguide 110, the opposing sides of adjacent layers may be bonded by known silicon fusion processes to form a waveguide having a desired thickness. For further example, for a titanium waveguide 110, the edges of adjacent layers may be bonded by known laser welding techniques to form a waveguide having a desired thickness. Composite structures, such as a laminated silicon-glass-silicon waveguide, may be bonded by related processes such as anodic bonding. For sake of clarity in this discussion and the appended claims, the term "end" will be understood as referring to a longitudinal boundary, or a surface representing such a boundary; the term "edge" will be understood as referring to a lateral boundary, or surface representing such boundary, in a direction within the plane of the waveguide; and the term "side" will be understood as referring to a lateral boundary, or surface representing such a boundary, in a direction perpendicular to the plane of the waveguide.

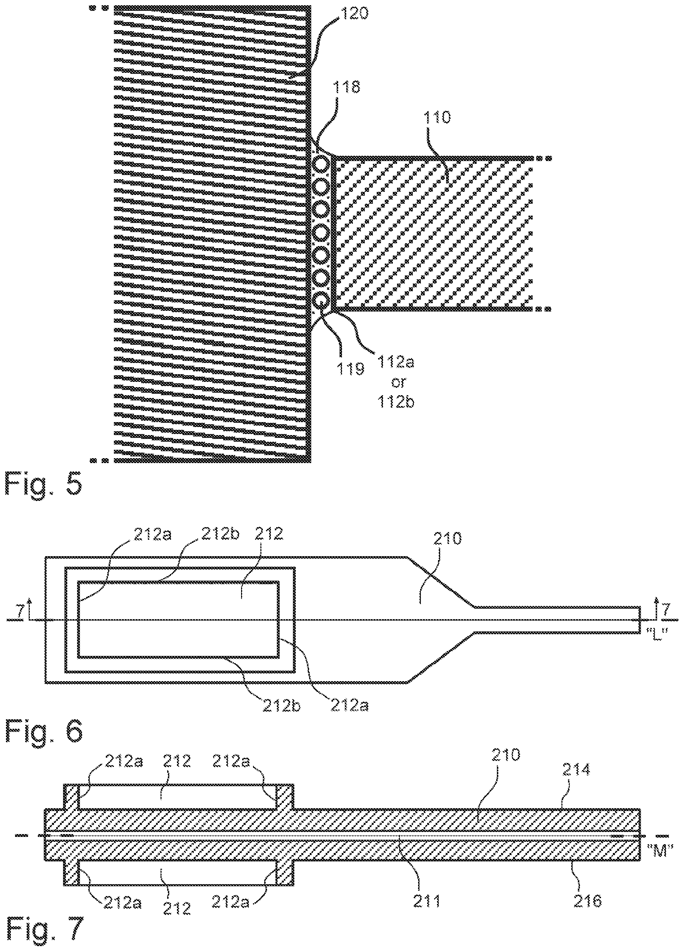

The ultrasonic core 100 also includes a transducer element 120. The transducer element is preferably formed from a lead-free piezoelectric material, such as barium titanate, or a magnetostrictive material, such as nickel or "GALFENOL" (gallium-iron alloys marketed by ETREMA Products, Inc. of Ames, Iowa), so that the ultrasonic surgical instrument may be both inexpensive enough to be employed as a single use device and suitable for disposal as ordinary medical waste, as opposed to lead-bearing hazardous waste. Other transducing materials, including ceramic PZT materials and electrostrictive materials, as well as single crystal materials, may also be used. As shown in Figures 4 and 5, the transducer element 120 may include a plurality of discrete transducing subelements configured as a "Langevin stack." However, it will be understood that the transducer element 120 may instead be a unitary transducer element like those illustrated in other figures.

As detailed in Figures 1 and 2, the planar waveguide 110 defines an aperture 112 extending from a first side 114 of the waveguide 110 to a second, opposite side 116 of the waveguide. The aperture 112 may be formed by laser cutting the waveguide 110, or through alternate methods such as water-jet cutting, depending upon the materials involved. As detailed in Figures 3 and 4, the transducer element 120 is sized and shaped to substantially conform to the size and shape of the aperture 112 (with respect to a length between ends and a length between edges, but not necessarily a depth or thickness between sides), and may be secured within the aperture 112 by a glue layer 118 disposed between the transducer element 120 and opposite walls of the aperture 112. In a first expression of the first embodiment, suitable for use in a device employing a longitudinal mode of vibration, the glue layer 118 may be disposed at opposite end walls 112a of the aperture 112. In a second expression of the first embodiment, suitable for use in a device employing a lateral mode of vibration, the glue layer 118 may be disposed at opposite edge walls 112b of the aperture 112. Preferably, the glue layer 118 is disposed about the entire periphery of the aperture 112 so as to completely secure the transducer element 120 within it. Disposition about the entire periphery of the aperture 112 will advantageously support the transmission of longitudinal, lateral, and torsional modes of vibration to the planar waveguide 110.

In a variation of the first embodiment, detailed in Figure 5, the glue layer 118 may include a plurality of rigid beads 119. The rigid beads 119 serve to increase the apparent modulus of elasticity of the glue layer 118 by interposing a substantially more rigid structure between the end walls 112a, edge walls 112b, and/or periphery of the aperture 112 and the ends, edges, and/or periphery of the transducer element 120. The glue of the glue layer 118 then serves as a matrix to substantially fix the position of the rigid beads 119 between respective portions of the aperture 112 and the transducer element 120. In a first expression of the embodiment, the rigid beads 119 may be non-conductive glass beads. In a second expression of the embodiment, the rigid beads 119 may be conductive beads, such as glass beads coated with a metallic layer, metallic beads, or the like. In the second expression, electrical power may be transferred between the planar waveguide 110 and the transducer element via the rigid beads 119, with the beads serving as a hot pole and/or a ground pole depending upon the particular configuration of the glue layer 118 (*e.g.,* disposed only at opposite walls of the aperture 112 so as to be configurable as two poles, or about the entire periphery of the aperture 112 so as to be configurable as a single pole). In either expression of the embodiment, the glue of the glue layer 118 may be conductive and serve as a as a hot pole and/or a ground pole apart from, or as a complement to, the rigid beads 119. Preferably, the rigid beads 119 are sized to form a single layer between the walls of the aperture 112 and the transducer element 120. Preferably, the rigid beads 119 make up between 10 percent and 65 percent, by volume, of the glue layer 118.

In contrast to a planar waveguide having a transducer element glued to a side of the waveguide, where movement is transmitted between the transducer element and the waveguide through an "elongation-propagation"-type action, the structures of the first embodiment provide a transducer element 120 embedded within the waveguide 110, permitting movement to be transmitted between them in a "push-pull"-type action. This change in transmission characteristics enhances the coupling and coupling efficiency between the structures by changing the principal forces acting across the bonding layer from shear forces to compressive forces, as well as permitting the use of improved bonding materials. Also, in contrast to a planar waveguide having transducer elements glued to opposite sides of the waveguide, the structures of the first embodiment eliminate the need to precisely align opposing transducer elements (in order to avoid undesired modes of vibration).

A second embodiment is shown in Figures 6-10. The second embodiment is an ultrasonic core 200 for an ultrasonic surgical instrument which includes a similar longitudinally elongated, generally planar waveguide 210 and a similar transducer element 220. As detailed in Figures 6 and 7, the planar waveguide 210 defines at least one aperture 212 extending from a first side 214 of the waveguide 210 toward a medial plane of the waveguide "M." However, in contrast to the first embodiment, the aperture is a blind or closed-ended aperture 212 as opposed to an open-ended aperture 112. In many devices, such as the exemplary devices discussed below, the planar waveguide 210 also defines an opposing aperture 212 extending from a second, opposite side 216 of the waveguide toward the medial plane "M" and/or additional apertures 212 positioned along a longitudinal axis of the waveguide "L." Each aperture 212 may be formed by machining or chemically etching the waveguide 210. Additionally or alternately, where more substantial transducer element thicknesses are required, the waveguide 210 may be a laminated structure including a plurality of planar layers. A principal layer or layers may constitute the transduction and resonator portions of the waveguide 210 and, optionally, a truncated auxiliary layer or layers may define the sides of the aperture 212. Each aperture 212 may then be formed by cutting, machining, and/or chemically etching one or more layers, either individually or in combination. As indicated earlier, the various layers may be bonded by silicon fusion processes, anodic bonding processes, laser welding processes, and other known techniques appropriate for use with the waveguide material(s).

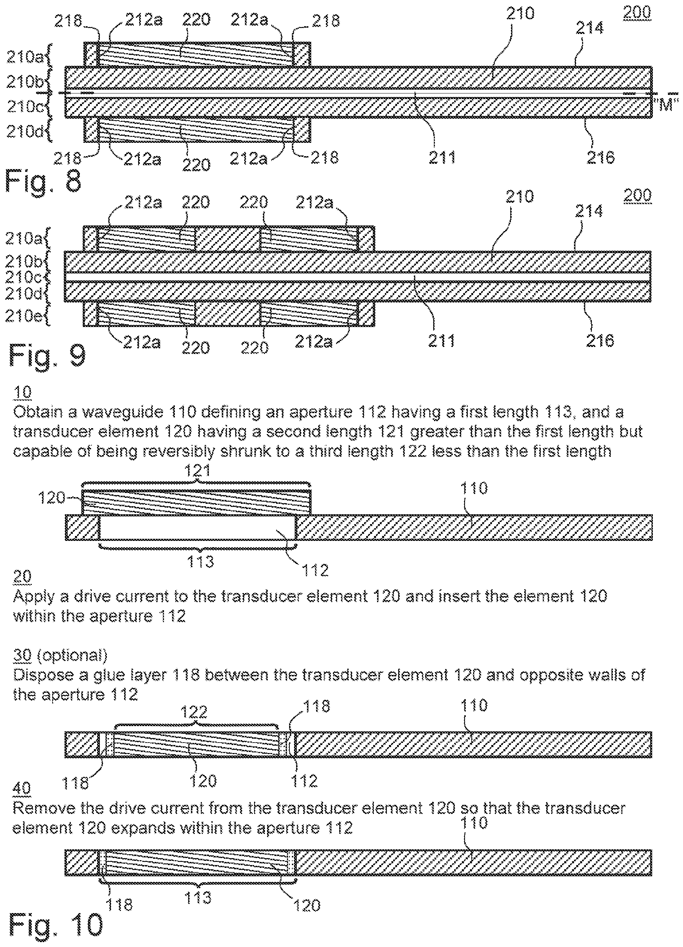

As detailed in Figure 8, each transducer element 220 is sized and shaped to substantially conform to the size and shape of its corresponding aperture 212, and may be secured within that aperture 212 by a glue layer 218 (not specifically shown) disposed between the transducer element 220 and opposite walls of the aperture 212. The transducer element 220 may also be secured to the closed end of the aperture 212 by glue layer 218, or by an alternate bonding material serving to temporarily position and secure the transducer element within the aperture 212 prior to introduction of the glue layer 218. In expressions of the second embodiment, the glue layer 218 may be disposed at the ends 212a of the aperture 212 and/or the edges 214b (not shown) of the aperture 212. Preferably, the glue layer 218 is disposed about the entire periphery of the aperture 212 so as to completely secure the transducer element 220 within it. Disposition about the entire periphery of the aperture 212 will advantageously support longitudinal, lateral, and torsional modes of vibration transmission to the planar waveguide 210. In a variation of the second embodiment, the glue layer 218 may include a plurality of rigid beads 219 (not illustrated for sake of clarity) like those described in the first embodiment and/or conductive components like those described in the first embodiment.

In contrast to a planar waveguide having transducer elements glued to opposite sides of the waveguide, the structures of the second embodiment provide transducer elements 220 at least partially embedded within the waveguide 210 itself. Each aperture 212 serves to positively locate a corresponding transducer element 220 with respect to an opposing transducer element disposed across the medial plane and/or a serial transducer element disposed at another predetermined (typically, longitudinal) location, and may be precisely positioned with respect to the waveguide 210 during manufacturing of the waveguide out of, for example, a silicon wafer or a titanium sheet. This change in structural characteristics reduces the difficulty in precisely aligning opposing transducer elements across the medial plane "M" in order to avoid undesired modes of vibration. This change in structural characteristics also reduces the difficulty in precisely positioning serial transducer elements at predetermined locations, e.g., at nodes of a desired mode of vibration, in order to avoid undesired modes of vibration and/or destructive interference between the elements of the series.

In a first example of the second embodiment, shown in Figure 8, the waveguide 210 is a laminated structure including a plurality of planar layers 210a, 210b, etc. of material. Two adjoining principal layers, 210b and 210c, define a longitudinal channel or other internal void which may serve, for example, as an internal lumen 211. Two truncated auxiliary layers, 210a and 210d, each define a blind aperture 212, with the apertures being disposed in mutual opposition across the medial plane "M." A pair of opposing transducer elements 220 are disposed within the apertures 212 and secured by glue layers 218 as described above. The first example consequently provides a structure suitable for use as an ultrasonic surgical scalpel which may be cooled by an irrigation fluid pumped through the internal lumen 211 in order to prevent the cauterization of dissected patient tissue.

In a second example of the second embodiment, shown in Figure 9, the waveguide 210 is a laminated structure including a plurality of planar layers 210a, 210b, etc. of material. Three adjoining principal layers, 210b, 210c, and 210d, define a longitudinal channel or other internal void which may serve, for example, as an internal lumen 211. In contrast to the first example, where the principal layers would be etched or otherwise machined to include partially penetrating voids defining the internal lumen 211, only the central principal layer 210c must be cut, machined, or otherwise shaped to provide a channel or void space, leaving the adjoining principal layers 210b and 210d to serve as the sides of the internal lumen 211. Two truncated auxiliary layers, 210a and 210e, each at least partially define a plurality of blind apertures 212 serially disposed along the longitudinal axis of the device, with the pluralities being disposed in mutual opposition across the medial plane "M." The adjoining principal layers, 210b and 210d, may further partially define the respective apertures 212 so as to provide reference points for locating the truncated auxiliary layers, 210a and 210e, during lamination of the various layers. Opposing transducer elements 220 are disposed within the apertures 212 and optionally secured by glue layers as described above. The second example provides a structure suitable for use as a cooled ultrasonic surgical scalpel, but also efficiently increases the amplitude of displacement of the waveguide and end effector by enabling the precise positioning of multiple smaller transducer elements in series. Those of skill in the art will appreciate that examples of the first embodiment may similarly employ a plurality of open-ended apertures 112 serially disposed along the longitudinal axis of the device to provide improved ultrasonic surgical scalpels and similar instruments.

As illustrated in Figure 10, in a method of assembling the devices of the first and second embodiments, the transducer element(s) 120, 220 may be sized and shaped to substantially conform to the size and shape of the aperture(s) 112, 212 after inversely powering the transducer element to shrink the length of the transducer element to a length which is shorter than a corresponding length of the aperture. In step 10, one obtains a longitudinally elongated, generally planar waveguide 110 defining an aperture 112 having a first length 113, and a transducer element 120 having a second length 121 greater than the first length 113 but capable of being reversibly shrunk to a third length 122 less than the first length 113 upon application of a drive current. In step 20, one applies the drive current to the transducer element 120 and inserts the transducer element 120 within the aperture 112. In optional step 30, one may dispose a glue layer 118 between the transducer element 120 and opposite walls of the aperture 112. As discussed above, such a glue layer 118 may include a plurality of rigid beads 119 which may serve to increase the apparent modulus of elasticity of the glue layer 118 and/or to ensure a constant glue layer thickness between the transducer element 120 and the opposite walls of the aperture 112. In step 40, one removes the drive current from the transducer element 120 so that the transducer element 120 expands within the aperture 112. Upon completion, the transducer element 120 is compressionally secured within and preconstrained by the opposite walls of the aperture 112. This preconstraint serves to increase the amplitude of displacement of the waveguide and end effector.

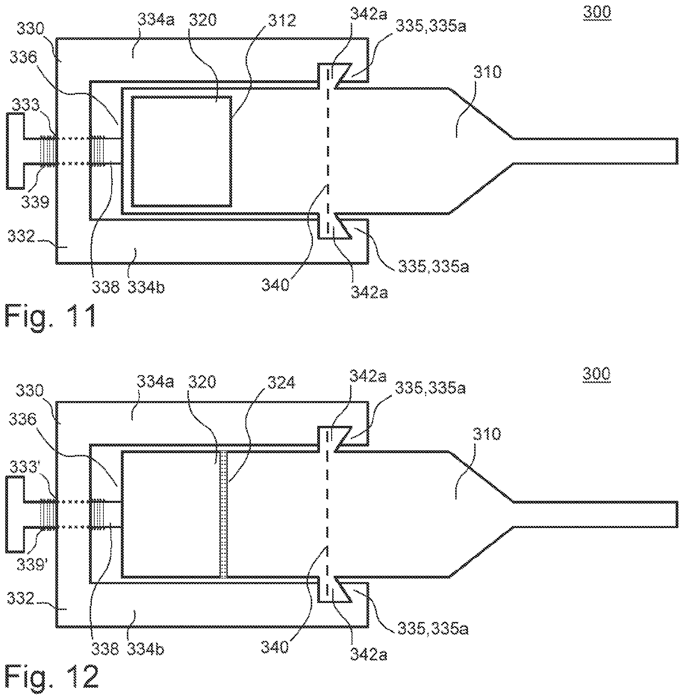

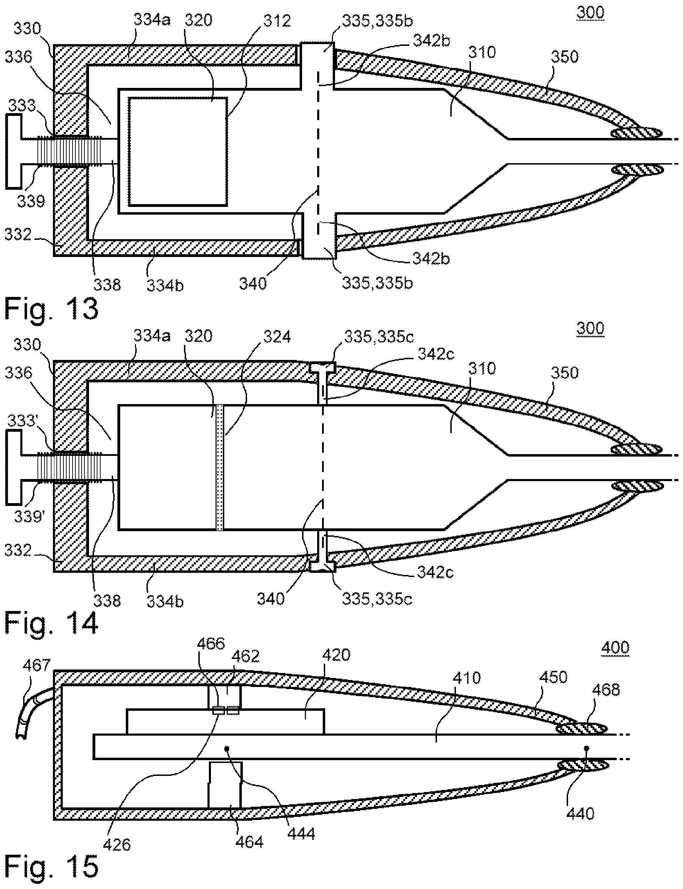

A third embodiment is shown in Figures 11 and 13. The third embodiment is an ultrasonic core 300 for an ultrasonic surgical instrument which includes a longitudinally elongated, generally planar waveguide 310, a transducer element 320 secured to the waveguide 310, and a clamp mechanism 330. The waveguide 310 may include an aperture 312, as shown in Figure 11, with the transducer element 320 sized and shaped to substantially conform to the size and shape of the aperture 312 and secured to opposite walls of the aperture 312. However, the transducer element 320 may alternately be secured to the proximal end of the waveguide 310, as shown in Figure 12, by a bonding material 324, which is not a part of the invention.

The clamp mechanism 330 may include a base 332 disposed proximally from the proximal end of the waveguide 310, a pair restraining arms 334a, 334b projecting distally from the base 332 and configured so as to mutually oppose one another across a channel 336 housing the waveguide 310, and a clamp arm 338 projecting distally from the base 332 between the pair of restraining arms 334a , 334b. Those of skill in the art will appreciate that the clamp mechanism may be an internal component of a handpiece for an ultrasonic surgical instrument, as shown in Figures 11 and 12, or be a unitary or integrated part of a handpiece housing 350 surrounding the transducer element 320, as shown in Figures 13 and 14. The base 332 and the clamp arm 338 may be mechanically engaged with one another so as to permit the distal end of the clamp arm 338 to be adjustably and securely positioned within the channel 336. For example, the base 332 may include an aperture 333 and the clamp arm 338 may include a sawtooth-ribbed section 339 enabling a ratchet-like advancement of the clamp arm 338 relative to the base 332. For further example, the base 322 may include a threaded aperture 333' (not specifically shown) and the clamp arm 338 may include a threaded section 339' (not specifically shown) enabling a screw-like advancement of the clamp arm 338 relative to the base 332. In other examples, the base 332 may include an aperture, channel, or groove, and the clamp arm may be adhered, soldered, or welded into the aperture, channel, or groove while the clamp arm 338 is secured to preconstrain the transducer element 120.

The restraining arms 334a, 334b each include a mount 335 which engages the waveguide 310 at a node 340 positioned distally from the transducer element 320. In a first expression of the third embodiment, shown in Figures 11 and 12, each mount includes a hook 335a, and the waveguide 310 includes complementary hooks 342a disposed proximate the node 340 and engaging the mount hooks. In a second expression of the third embodiment, shown in Figure 13, each mount includes a slot 335b, and the waveguide includes projections 342b extending outwardly from the edges of the waveguide 310 proximate the node 340 and engaging the mount slots. In a third expression of the third embodiment, shown in Figure 14, each mount includes a pin or screw 335c projecting into the channel 336, and the waveguide includes sockets 342c extending inwardly from the edges of the waveguide 310 proximate the node 340 and engaging the mount pins or screws.

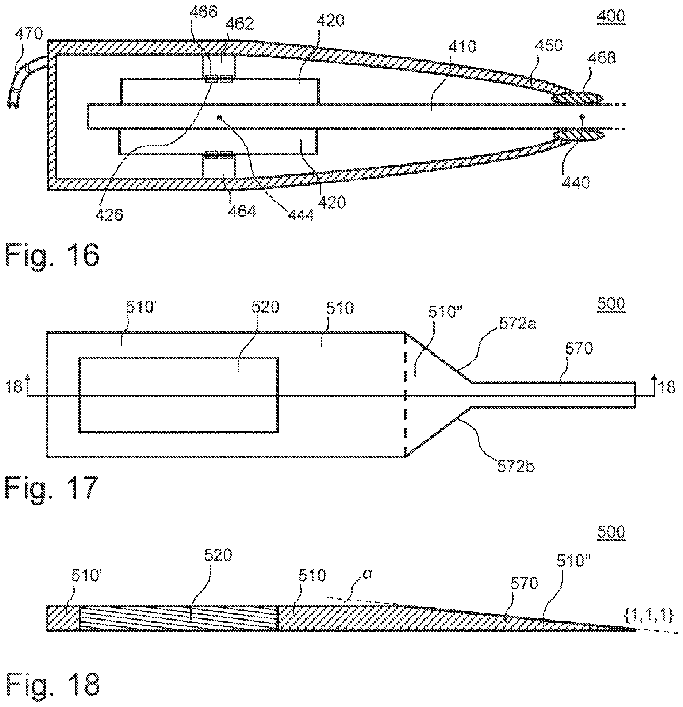

an ultrasonic handpiece is shown in Figures 15 and 16. The ultrasonic handpiece 400 for an ultrasonic surgical instrument includes a longitudinally elongated, generally planar waveguide 410, a transducer element 420 secured to the waveguide 410, a housing 450 surrounding at least the transducer element 420, and a clamp mechanism 460 secured to the housing 450 proximate the transducer element 420 and engaging the transducer element at a transducer node 444. The clamp mechanism 460 may principally secure the planar waveguide 410 and transducer element 420 within the housing 450 by applying a transverse compressional force to these structures, but may also engage a groove, aperture, or other locating structure with complementary structure such as a tongue or post. The complementary structure may be at least partially elastomeric (e.g., include an elastomeric tip or cushion) so as to vibrationally isolate the housing 450 from the transducer element 420. The clamp mechanism 460 and the transducer element 420 include complementary electrical contacts 466, 426 for applying a drive current to the transducer element 420. The clamp mechanism electrical contacts 466 may be electrically connected to a remote ultrasound generator via a handpiece cable 467, and the transducer element electrical contacts 426 may be electrically connected to a plurality of electrodes 428 (not shown for sake of clarity) disposed upon and within the transducer element 420.

In an example shown in Figure 15, a transducer element 420 is secured to the first side 414 of the waveguide 410 without an opposing transducer element being secured to the second, opposite side 416 of the waveguide. The clamp mechanism 460 may include a first clamp arm 462 engaging the transducer element 420 at the transducer node 444 and a second clamp arm 464 engaging the waveguide 410, specifically the second, opposite side 416 of the waveguide, at the transducer node 444. The clamp mechanism electrical contacts 466 are then disposed on the first clamp arm 462. A joint 468, such as an O-ring or similar generally annular elastomeric part, may engage the waveguide 410 at a node 440 positioned distally from the transducer element 420 to increase the stiffness of the waveguide 410 and to prevent contaminants from entering the housing 450.

In a second example shown in Figure 16, a first transducer element 420a is secured to the first side 414 of the waveguide 410 and a second, opposing transducer element 420b is secured to the second, opposite side 416 of the waveguide. The clamp mechanism 460 may include a first clamp arm 462 engaging the first transducer element 420a at the transducer node 444 and a second clamp arm 464 engaging the second transducer element 420b at the transducer node 444. The clamp mechanism electrical contacts 466 may then be disposed on both the first clamp arm 462 and the second clamp arm 464 for engagement with the complementary electrical contacts 426 of the respective transducer elements. Again, a joint 468, such as an O-ring or similar generally annular elastomeric part, may engage the waveguide 410 at a node 440 positioned distally from the transducer elements 420a and 420b to increase the stiffness of the waveguide 410 and to prevent contaminants from entering the housing 450.

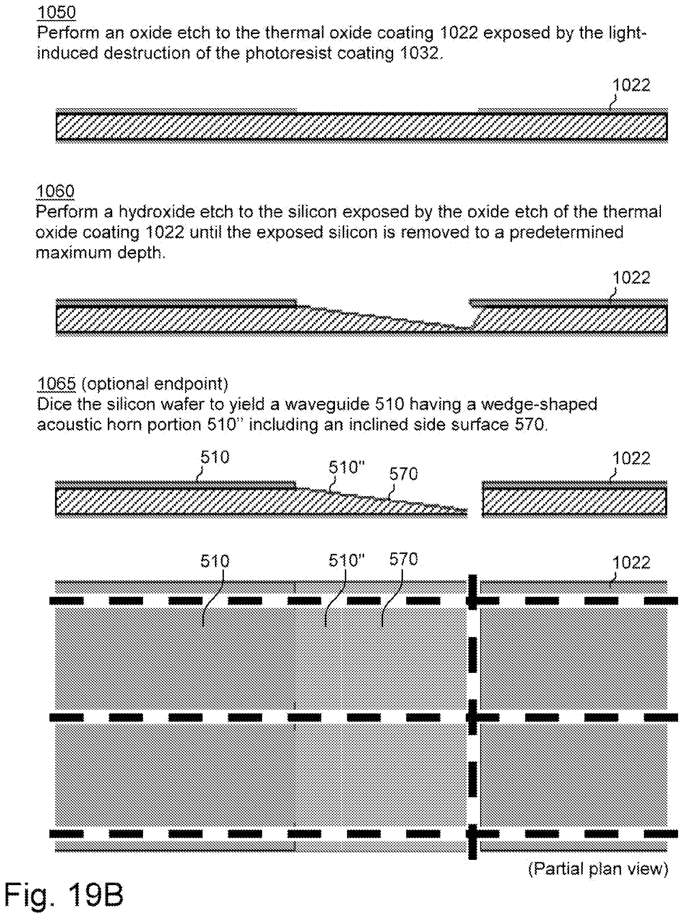

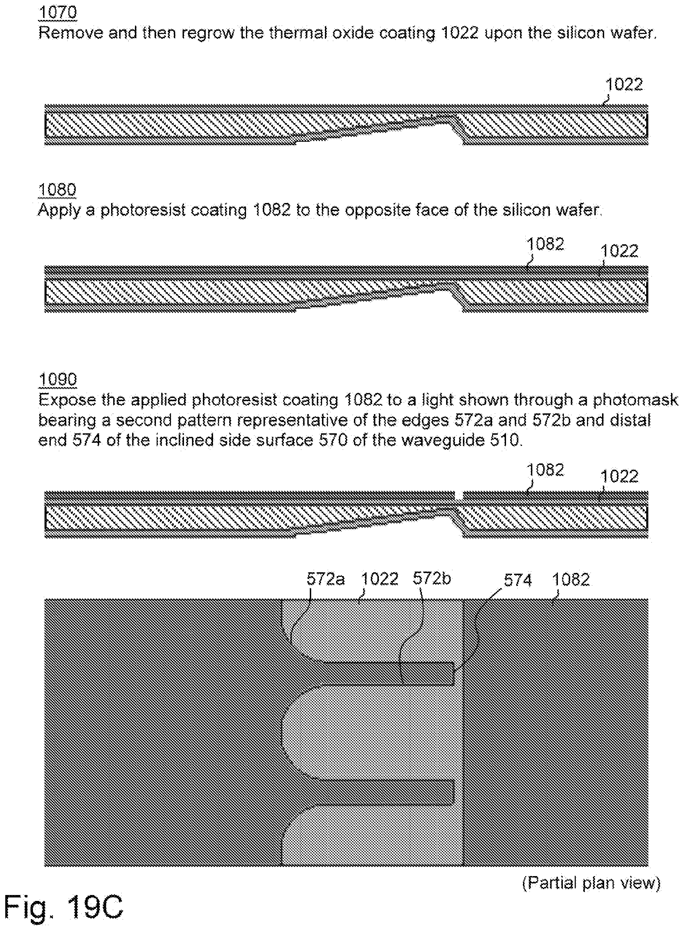

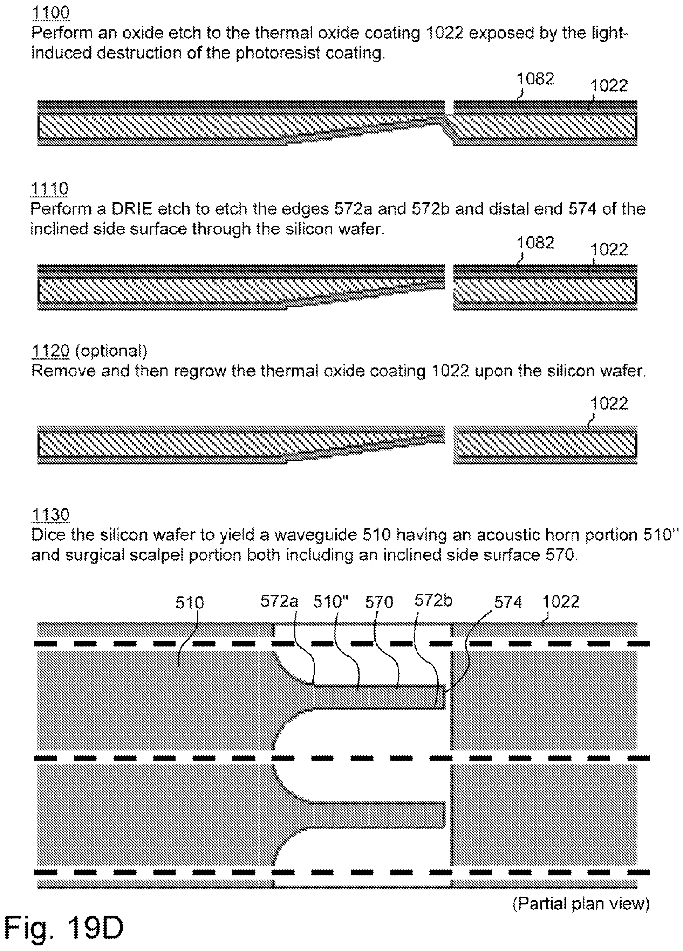

A fourth embodiment is shown in Figures 17 and 18. The fourth embodiment is an ultrasonic core 500 which includes a longitudinally elongated, generally planar silicon waveguide 510 having a generally planar transduction portion 510', with at least one transducer element 520 secured to the generally planar transduction portion 510', and a wedge-shaped acoustic horn portion 510" including an inclined side surface 570. The fourth embodiment is characterized in that the inclined side surface 570 is oriented along the {1,1,1} crystallographic plane of the silicon material. The edges of the inclined side surface, 572a and 572b, may also converge toward a central longitudinal axis of the waveguide "L" so as to provide a three-dimensionally varying acoustic horn. In a first example of the fourth embodiment, shown in Figures 17 and 18, the edges of the inclined side surface 572a and 572b may linearly converge toward the central longitudinal axis "L" in the acoustic horn portion 510". In a second example of the fourth embodiment, partially shown in Figure 19C, the edges of the inclined side surface 572a and 572b may curvilinearly converge toward the central longitudinal axis "L" in the acoustic horn portion 510".

In a variation of the fourth embodiment, the acoustic horn portion 510" may include a unitary surgical scalpel portion.

Although the following discussion focuses upon a method of manufacturing a waveguide for the ultrasonic core of the fourth embodiment, it is expressly contemplated that the generally planar transduction portion 510' may take the form of any one of the first through third embodiments and combinations thereof, and that the waveguide 510 of the fifth embodiment may be a laminated structure having a pair of mutually opposing principal layers, each including a wedge-shaped acoustic horn portion 510", adjoined such that the inclined side surfaces 570 of the respective layers are disposed on opposite sides of the waveguide 510. For sake of clarity in this discussion and the appended claims, the term "ordered" will be understood as referring to a set of steps that, with respect to each other, are carried out in the stated order, but shall not be interpreted as precluding or excluding the possibility of some other step or steps being performed before, during, or after a recited step nor of some other step of steps being performed between recited steps. Rather, the performance of each recited step serves as a prerequisite to performance of the next.

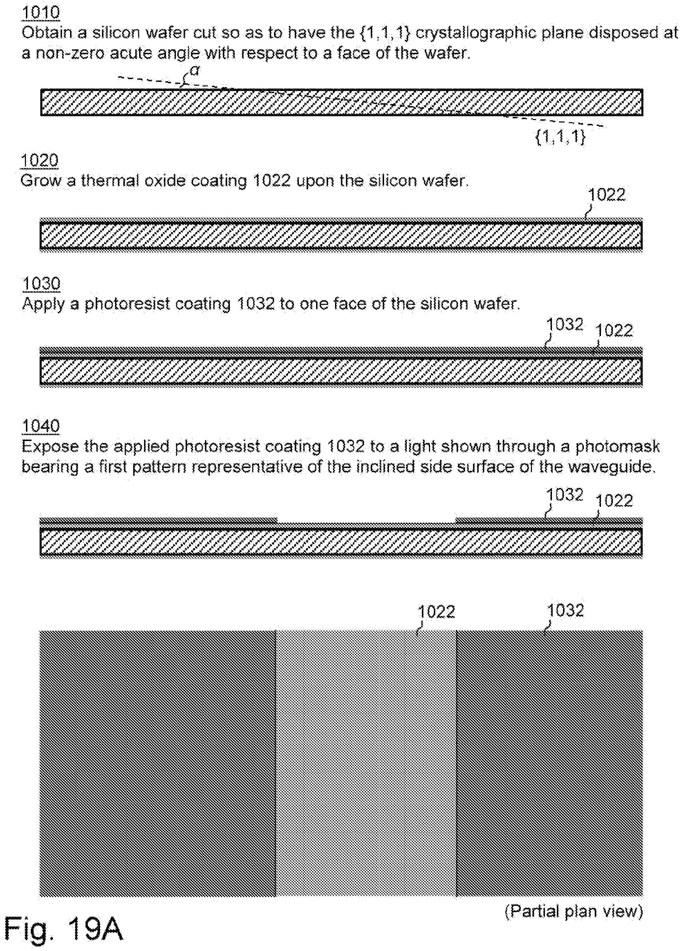

As illustrated in Figures 19A through 19D, in a method of manufacturing a silicon waveguide for an ultrasonic surgical instrument, a silicon wafer is serially coated, etched, and diced in a number of ordered steps so as to manufacture at least one waveguide 510 having an inclined side surface 570 oriented along a {1,1,1} crystallographic plane of the silicon material. In step 1010, one obtains a silicon wafer cut so as to have the {1,1,1} crystallographic plane disposed at a non-zero acute angle with respect to a face of the wafer. Preferably, the {1,1,1} crystallographic plane is disposed at an angle α of between 1 degrees and 10 degrees with respect to this face. In step 1020, one grows a thermal oxide coating 1022 upon the silicon wafer; in step 1030, one applies a photoresist coating 1032 to one face of the silicon wafer; and in step 1040, one exposes the applied photoresist coating 1032 to a light shown through a photomask bearing a first pattern representative of the inclined side surface 570 of the waveguide 510. In step 1050, one performs an oxide etch, such as a buffered hydrofluoric acid etch (BHF etch) or plasma etch, to the thermal oxide coating 1022 exposed by the light-induced destruction of the photoresist coating 1032, and then removes the residual photoresist coating 1032.

In step 1060, one performs a hydroxide etch, using etchants such as potassium hydroxide or tetramethylammonium hydroxide, to the silicon exposed by the oxide etch of the thermal oxide coating 1022. The silicon oriented along the {1,1,1} crystallographic plane of the material and having edges protected by the thermal oxide will be comparatively resistant to the etching process, whereas the silicon oriented along other crystallographic planes such as the {1,0,0} plane and having edges exposed to the etchant will be comparatively susceptible to the etching process. For example, in a KOH etching process the relative ratio of etching rates for the material in the {1,1,1} and {1,0,0} planes will be approximately 1:100, creating a V-like notch in the silicon material having a 'long' leg or side oriented along the {1,1,1} crystallographic plane and a 'short' leg or side where silicon material is being more rapidly removed. The hydroxide etch is performed until the exposed silicon is removed to a predetermined maximum depth, creating an inclined surface oriented along the {1,1,1} crystallographic plane of the silicon material. Preferably the predetermined maximum depth is just less than the depth of the silicon wafer so as to form an acoustic horn and unitary ultrasonic surgical scalpel projecting distally therefrom. Alternately, the predetermined maximum depth is a non-zero depth, substantially less than the depth of the silicon wafer, so as to form an acoustic horn providing a stud for the attachment of an ultrasonic end effector. In the latter instance, in step 1065, one may dice the silicon wafer to yield a waveguide 510 or waveguide layer having a wedge-shaped acoustic horn portion 510" including the inclined side surface 570. It will be appreciated that the silicon exposed by the hydroxide etching and, potentially, dicing operation may be converted to a thermal oxide coating so as to improve the strength of the acoustic horn portion. An ultrasonic end effector could later be glued to the distal end of the formed acoustic horn, or fused to the distal end of the formed acoustic horn by known silicon fusion processes, anodic bonding processes, or the like to yield an ultrasonic surgical instrument with an acoustic horn which tapers in both transverse dimensions, like the machined fore-end mass acoustic horns of conventional ultrasonic transducer assemblies.

In the former instance, where one seeks to manufacture a unitary ultrasonic surgical scalpel, further processing steps are performed. In step 1070, one removes and then regrows the thermal oxide coating 1022 upon the silicon wafer; and, in step 1080, one applies a photoresist coating 1082 to the opposite face of the silicon wafer. In step 1090, one exposes the applied photoresist coating to a light shown through a photomask bearing a second pattern representative of the edges 572a and 572b and distal end 574 of the inclined side surface 570 of the waveguide 510. In step 1100, one performs an oxide etch, such as a plasma etch, to the thermal oxide coating exposed by the light-induced destruction of the photoresist coating. In step 1110, one performs a DRIE etch to etch the edges 572a and 572b and distal end 574 of the inclined side surface 570 through the silicon wafer. Then, in step 1120, one optionally removes and then regrows the thermal oxide coating upon the silicon wafer so as to improve the strength and biocompatibility of the surgical scalpel portion; and, in step 1130, one dices the wafer to yield a waveguide 510 or waveguide layer having a wedge-shaped acoustic horn portion 510" and surgical scalpel portion both including the inclined side surface 570.

The wedge-shaped acoustic horn with inclined side surface enables the manufacturer to produce an acoustic horn which tapers in both transverse dimensions, like the machined fore-end mass acoustic horns of conventional ultrasonic transducer assemblies, and to deliver enhanced ultrasonic gain due to the narrowing of the waveguide material along the inclined side surface. In constructions like that of the fourth embodiment, where the wedge-shaped distal portion of the waveguide includes an acoustic horn portion and a unitary surgical scalpel portion, both portions may include the inclined side surface. The inclined side surface consequently serves to provide enhanced ultrasonic gain to the surgical scalpel and to provide a very sharp distal-most edge, as well as to provide a smooth transition from the acoustic horn portion to the surgical scalpel portion. The method of manufacturing the waveguide takes advantage of the fact that silicon etching processes can be anisotropic, with the structures of the crystal planes being etched at different rates so as to preferentially remove silicon across the {1,0,0} and {1,1,0} crystallographic planes while intrinsically forming a surface aligned with the {1,1,1} crystallographic plane. Thus, an essentially monolithic essentially monolithic transducer-supporting waveguide can be manufactured to provide superior acoustic gain to a distal end without requiring expensive and complete three dimensional machining equipment and techniques, and ultrasonic handpieces employing such waveguides can be manufactured with smaller ultrasonic cores for a desired amount of end effector displacement.

While aspects of the invention have been illustrated by the description of several embodiments, it is not the intention of the applicant to restrict or limit the scope of the appended claims to such detail. Numerous variations, changes, and substitutions will occur to those skilled in the art without departing from the scope of the invention. Moreover, the structure of each element associated with the present invention can be alternatively described as a means for providing the function performed by the element. Accordingly, it is intended that the invention be limited only by the scope of the appended claims.

## Claims

1. An ultrasonic core for an ultrasonic surgical instrument, the ultrasonic core comprising:
a longitudinally elongated, generally planar waveguide (110) having an aperture (112) extending from a first side of the waveguide (110) toward a medial plane of the waveguide (110); and
a transducer element (120) secured to opposite walls of the aperture (112), wherein the transducer element (120) is sized and shaped to substantially conform to the size and shape of the aperture (112),
wherein the transducer element (120) is secured to the opposite walls of the aperture (112) by a glue layer (118) disposed between the transducer element (120) and the opposite walls of the aperture (112),
**characterized in that**
the glue layer (118) includes a plurality of rigid beads (119).

2. The ultrasonic core of claim 1, wherein the glue layer (118) is disposed at the ends (112a) of the aperture.

3. The ultrasonic core of claim 2, wherein the glue layer (118) is disposed about the entire periphery of the aperture (112).

4. The ultrasonic core of claim 1, wherein the rigid beads (119) are glass beads.

5. The ultrasonic core of claim 1, wherein the aperture (112) extends from the first side of the waveguide (110) to a second, opposite side of the waveguide (110).

6. The ultrasonic core of claim 1, wherein the aperture (112) is a blind aperture having a closed end.

7. The ultrasonic core of claim 6, wherein the transducer element (120) is secured to the closed end of the aperture (112) by the glue layer (118).

8. The ultrasonic core of claim 1, wherein the waveguide (110) is a laminated structure including a plurality of planar layers.

9. A method of assembling an ultrasonic core including a longitudinally elongated, generally planar waveguide (110), the method comprising the steps of:
obtaining a longitudinally elongated, generally planar waveguide (110) defining an aperture (112) having a first length, and a transducer element (120) having a second length greater than the first length but capable of being reversibly shrunk to a third length less than the first length upon application of a drive current;
applying the drive current to the transducer element (120) and inserting the transducer element (120) within the aperture (112);
disposing a glue layer (118) including a plurality of rigid beads (119) between the transducer element (120) and opposite walls of the aperture (112) prior to removing the drive current from the transducer element (120) so that the transducer element (120) expands within the aperture (112),
whereby the transducer element (120) is compressionally secured within and preconstrained by the opposite walls of the aperture (112).

10. The method of claim 9, wherein the glue layer (118) is disposed at the ends of the aperture (112).

11. The method of claim 10, wherein the glue layer (118) is disposed about the entire periphery of the aperture (112).

12. The method of claim 9, wherein the rigid beads (119) are glass beads.

13. The ultrasonic core of claim 1, further comprising:
a clamp mechanism (330) including a base (332) disposed proximally from the proximal end of the waveguide (310), a pair of restraining arms (334a, 334b) projecting distally from the base (332) and configured so as to mutually oppose one another across a channel (336) housing the waveguide (310), and a clamp arm (338) projecting distally from the base (332) between the pair of restraining arms (334a, 334b),
wherein the base (332) and the clamp arm (338) are mechanically engaged with one another so as to permit distal end of the clamp arm (338) to be securely positioned within the channel (336); and
wherein each restraining arm (334a, 334b) includes a mount (335) which engages the waveguide (310) at a node (340) positioned distally from the transducer element (320).

14. The ultrasonic core of claim 1, wherein:
the waveguide (510) is a silicon waveguide having a generally planar transduction portion (510'), with the transducer element (520) secured to the generally planar transduction portion (510'), and a wedge-shaped acoustic horn portion (510") including an inclined side surface (570),
wherein the inclined side surface (570) is oriented along the {1,1,1} crystallographic plane of the silicon material.

## Patentansprüche

1. Ultraschallkern für ein chirurgisches Ultraschallinstrument, wobei der Ultraschallkern umfasst:
einen in Längsrichtung langgestreckten, allgemein planaren Wellenleiter (110) mit einer Öffnung (112), die sich von einer ersten Seite des Wellenleiters (110) zu einer Mittelebene des Wellenleiters (110) erstreckt; und
ein Wandlerelement (120), das an gegenüberliegenden Wänden der Öffnung (112) befestigt ist, wobei das Wandlerelement (120) bemessen und geformt ist, um der Größe und Form der Öffnung (112) im Wesentlichen zu entsprechen,
wobei das Wandlerelement (120) durch eine Klebstoffschicht (118), die zwischen dem Wandlerelement (120) und den gegenüberliegenden Wänden der Öffnung (112) angeordnet ist, an den gegenüberliegenden Wänden der Öffnung (112) befestigt ist,
**dadurch gekennzeichnet, dass** die Klebstoffschicht (118) eine Vielzahl von starren Kügelchen (119) umfasst.

2. Ultraschallkern gemäß Anspruch 1, wobei die Klebstoffschicht (118) an den Enden (112a) der Öffnung angeordnet ist.

3. Ultraschallkern gemäß Anspruch 2, wobei die Klebstoffschicht (118) um den gesamten Umfang der Öffnung (112) angeordnet ist.

4. Ultraschallkern gemäß Anspruch 1, wobei die starren Kügelchen (119) Glaskügelchen sind.

5. Ultraschallkern gemäß Anspruch 1, wobei sich die Öffnung (112) von der ersten Seite des Wellenleiters (110) zu einer zweiten, gegenüberliegenden Seite des Wellenleiters (110) erstreckt.

6. Ultraschallkern gemäß Anspruch 1, wobei sich die Öffnung (112) eine blinde Öffnung mit einem geschlossenen Ende ist.

7. Ultraschallkern gemäß Anspruch 6, wobei das Wandlerelement (120) durch die Klebstoffschicht (118) an dem geschlossenen Ende der Öffnung (112) befestigt ist.

8. Ultraschallkern gemäß Anspruch 1, wobei der Wellenleiter (110) eine laminierte Struktur ist, die eine Vielzahl von planaren Schichten aufweist.

9. Verfahren zum Zusammensetzen eines Ultraschallkerns, der einen in Längsrichtung langgestreckten, allgemein planaren Wellenleiter (110) umfasst, wobei das Verfahren die Schritte umfasst:
Erhalten eines in Längsrichtung langgestreckten, allgemein planaren Wellenleiters (110), der eine Öffnung (112) mit einer ersten Länge definiert, und eines Wandlerelements (120), das eine zweite Länge aufweist, die größer als die erste Länge ist, aber fähig ist, bei Anlegen eines Treiberstroms reversibel auf eine dritte Länge geschrumpft zu werden, die kleiner als die erste Länge ist;
Anlegen des Treiberstroms an das Wandlerelement (120) und Einführen des Wandlerelements (120) in die Öffnung (112);
Anordnen einer Klebstoffschicht (118), die eine Vielzahl von starren Kügelchen (119) enthält, zwischen das Wandlerelement (120) und gegenüberliegende Wände der Öffnung (112), bevor der Treiberstrom von dem Wandlerelement (120) gelöst wird, so dass das Wandlerelement (120) innerhalb der Öffnung (112) expandiert;
wodurch das Wandlerelement (120) durch Kompression innerhalb der gegenüberliegenden Wände der Öffnung (112) befestigt und davon vorgespannt wird.

10. Verfahren gemäß Anspruch 9, wobei die Klebstoffschicht (118) an den Enden der Öffnung (112) angeordnet ist.

11. Verfahren gemäß Anspruch 10, wobei die Klebstoffschicht (118) um den gesamten Umfang der Öffnung (112) angeordnet ist.

12. Verfahren gemäß Anspruch 9, wobei die starren Kügelchen (119) Glaskügelchen sind.

13. Ultraschallkern gemäß Anspruch 1, ferner umfassend:
einem Klemmmechanismus (330), umfassend eine Basis (332), die proximal bezogen auf das proximale Ende des Wellenleiters (310) angeordnet ist, ein Paar von Haltearmen (334a, 334b), die distal von der Basis (332) vorragen und so gestaltet sind, dass sie einander entlang eines Kanals (336) gegenüberliegen, der den Wellenleiter (310) aufnimmt, und einem Klemmenarm (338), der distal von der Basis (332) zwischen dem Paar von Haltearmen (334a, 334b) vorragt,
wobei die Basis (332) und der Klemmenarm (338) mechanisch miteinander in Eingriff stehen, um zu erlauben, dass das distale Ende des Klemmenarms (338) sicher innerhalb des Kanals (336) positioniert wird; und
wobei jeder Haltearm (334a, 334b) eine Halterung (335) aufweist, die in den Wellenleiter (310) an einem Knoten (340) eingreift, der distal bezogen auf das Wandlerelement (320) angeordnet ist.

14. Ultraschallkern gemäß Anspruch 1, wobei:
der Wellenleiter (510) ein Silicium-Wellenleiter mit einem allgemein planaren Wandlerabschnitt (510') ist, wobei das Wandlerelement (520) an dem allgemein planaren Wandlerabschnitt (510') befestigt ist, und einen keilförmigen Schallhornabschnitt (510"), der eine schräge Seitenfläche (570) aufweist,
wobei die schräge Seitenfläche (570) entlang der kristallographischen {1,1,1}-Ebene des Siliciummaterials ausgerichtet ist.

## Revendications

1. Noyau ultrasonique pour un instrument chirurgical ultrasonique, le noyau ultrasonique comprenant:
un guide d'ondes essentiellement planaire longitudinalement allongé (110) comportant une ouverture (112) qui s'étend à partir d'un premier côté du guide d'ondes (110) en direction d'un plan médial du guide d'ondes (110); et
un élément de transducteur (120) fixé à des parois opposées de l'ouverture (112), dans lequel l'élément de transducteur (120) est dimensionné et configuré de manière à correspondre sensiblement à la taille et à la forme de l'ouverture (112),
dans lequel l'élément de transducteur (120) est fixé aux parois opposées de l'ouverture (112) à l'aide d'une couche de colle (118) déposée entre l'élément de transducteur (120) et les parois opposées de l'ouverture (112),
**caractérisé en ce que** la couche de colle (118) comprend une pluralité de perles rigides (119).

2. Noyau ultrasonique selon la revendication 1, dans lequel la couche de colle (118) est déposée aux extrémité (112a) de l'ouverture.

3. Noyau ultrasonique selon la revendication 2, dans lequel la couche de colle (118) est déposée autour de la totalité de la périphérie de l'ouverture (112).

4. Noyau ultrasonique selon la revendication 1, dans lequel les perles rigides (119) sont des perles de verre.

5. Noyau ultrasonique selon la revendication 1, dans lequel l'ouverture (112) s'étend à partir du premier côté du guide d'ondes (110) jusqu'à un second côté opposé du guide d'ondes (110).

6. Noyau ultrasonique selon la revendication 1, dans lequel l'ouverture (112) est une ouverture aveugle qui présente une extrémité fermée.

7. Noyau ultrasonique selon la revendication 6, dans lequel l'élément de transducteur (120) est fixé à l'extrémité fermée de l'ouverture (112) par la couche de colle (118).

8. Noyau ultrasonique selon la revendication 1, dans lequel le guide d'ondes (110) est une structure stratifiée qui comprend une pluralité de couches planes.

9. Procédé d'assemblage d'un noyau ultrasonique comprenant un guide d'ondes essentiellement planaire longitudinalement allongé (110), le procédé comprenant les étapes suivantes:
obtenir un guide d'ondes essentiellement planaire longitudinalement allongé (110) définissant une ouverture (112) présentant une première longueur, et un élément de transducteur (120) présentant une seconde longueur plus grande que la première longueur mais capable d'être contractée de façon réversible à une troisième longueur plus petite que la première longueur lors de l'application d'un courant d'attaque;
appliquer le courant d'attaque à l'élément de transducteur (120) et insérer l'élément de transducteur (120) à l'intérieur de l'ouverture (112); et
déposer une couche de colle (118) comprenant une pluralité de perles rigides (119) entre l'élément de transducteur (120) et des parois opposées de l'ouverture (112) avant de supprimer le courant d'attaque de l'élément de transducteur (120) de telle sorte que l'élément de transducteur (120) se dilate à l'intérieur de l'ouverture (112),
dans lequel l'élément de transducteur (120) est fixé par compression à l'intérieur de l'ouverture (112) et est précontraint par les parois opposées de celle-ci.

10. Procédé selon la revendication 9, dans lequel la couche de colle (118) est déposée aux extrémités de l'ouverture (112).

11. Procédé selon la revendication 10, dans lequel la couche de colle (118) est déposée autour de la totalité de la périphérie de l'ouverture (112).

12. Procédé selon la revendication 9, dans lequel les perles rigides (119) sont des perles de verre.

13. Noyau ultrasonique selon la revendication 1, comprenant en outre:
un mécanisme de serrage (330) comprenant une base (332) déposée à proximité de l'extrémité proximale du guide d'ondes (310), une paire de bras de retenue (334a, 334b) faisant saillie en position distale à partir de la base (332) et configurés de manière à s'opposer mutuellement l'un l'autre en travers d'un canal (336) contenant le guide d'ondes (310), et un bras de serrage (338) faisant saillie en position distale à partir de la base (332) entre la paire de bras de retenue (334a, 334b),
dans lequel la base (332) et le bras de serrage (338) sont engagés de façon mécanique l'un avec l'autre de manière à permettre à une extrémité distale du bras de serrage (338) d'être positionnée fixement à l'intérieur du canal (336); et
dans lequel chaque bras de retenue (334a, 334b) comprend une monture (335) qui engage le guide d'ondes (310) au niveau d'un noeud (340) positionné de façon distale à partir de l'élément de transducteur (320).

14. Noyau ultrasonique selon la revendication 1, dans lequel:
le guide d'ondes (510) est un guide d'ondes en silicone qui présente une partie de transduction essentiellement plane (510') avec l'élément de transducteur (520) fixé à la partie de transduction essentiellement plane (510'), et une partie de pavillon acoustique en forme de coin (510") présentant une surface latérale inclinée (570), et
dans lequel la surface latérale inclinée (570) est orientée le long du plan cristallographique {1,1,1} de la matière de silicone.
